# FASCICULE DE BREVET EUROPEEN

(11) **EP 0 821 971 B1**
(45) Date de publication et mention de la délivrance du brevet: **12.03.2003**
(21) Numéro de dépôt: 97904460.9
(22) Date de dépôt: 19.02.1997
(51) Int. Cl.: A61L 9/12, A01M 1/20

(54) **SUPPORT-BOITE AMELIORE POUR PASTILLE DEGAGEANT DES VAPEURS ET DES EFFLUVES ODORANTES, DESINFECTANTES ET INSECTICIDES**
TRÄGERBEHÄLTER FÜR TABLETTEN, DIE DUFTENDE, DESINFIZIERENDE UND INSEKTIZIDE DÄMPFE UND AUSDÜNSTUNGEN ABGEBEN
IMPROVED SUPPORT-CONTAINER FOR TABLETS GIVING OFF ODORIFEROUS, DISINFECTANT AND INSECTICIDE VAPORS AND EFFLUVIA

(30) Priorité: 20.02.1996 ES 9600501 U
(43) Date de publication de la demande: 04.02.1998
(73) Titulaire: S.A. Margal, 08006 Barcelona (ES)
(72) Inventeur: FERNANDEZ MARTORELL, Ignacio, E-08006 Barcelona (ES)
(74) Mandataire: Canela Giménez, Teresa
(86) Numéro de dépôt international: ES9700035
(87) Numéro de publication internationale: WO97030738

## Description

La présente invention concerne une boîte support améliorée pour des plaquettes dégageant des vapeurs et des effluves odorants, désinfectants et insecticides.

Le demandeur est propriétaire du modèle d'utilité ES-U-289.919 ayant pour titre "Nouvelle boîte support pour des plaquettes dégageant des vapeurs et des effluves odorants, désinfectants et insecticides".

Pour simplifier l'opération d'assemblage des deux demi-boîtes emboîtées, on a eu l'idée de l'objet de la présente invention dans lequel l'opération d'emboîtement de l'élément male dans l'élément femelle est supprimée et á la place la liaison est réalisée par accrochage.

Pour avoir une interprétation correcte, on décrira ci-après un mode de réalisation pratique, á titre d'exemple non limitatif, de la nouvelle boîte support, accompagnée de dessins.

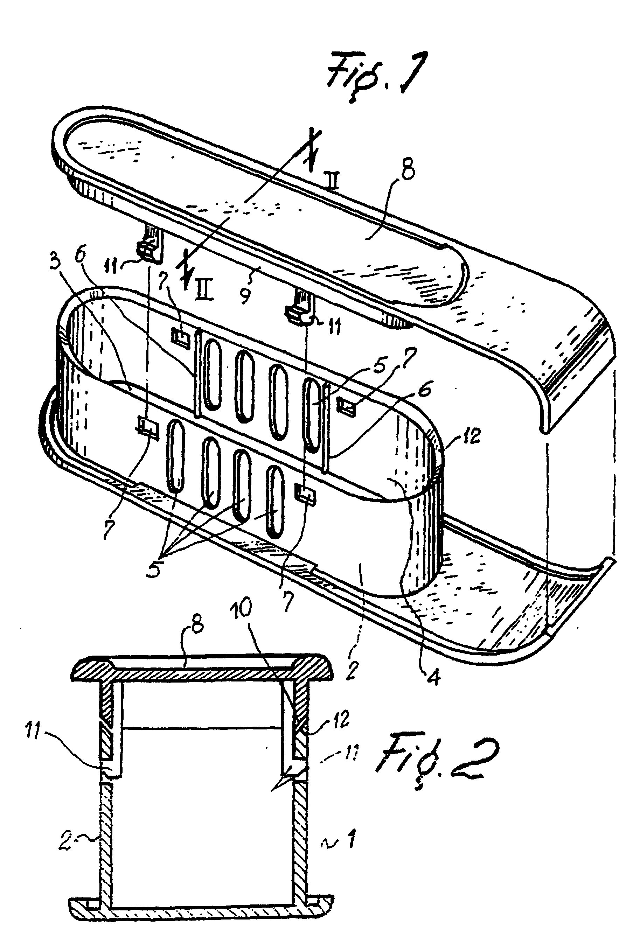

Sur la figure 1 est représentée la nouvelle boîte du type de celle du modèle d'utilité ES-U-289.919 du demandeur, vue en perspective, avec le couvercle séparé et aligné. Sur cette figure n'est pas représentée la bande continue de réglage de fermeture ou d'ouverture contrôlée pour la sortie des vapeurs diffusées par le corps, lui-même non représenté, de la plaquette interne émettant les vapeurs, afin de faire comprendre plus clairement les améliorations apportées par le nouvel accrochage de la boîte.

La figure 2 est une vue en coupe faite suivant la ligne II-II de la figure 1 et sur laquelle on peut voir le corps de la boîte avec son couvercle appliqué et accroché. Sur la figure 2 n'est cependant pas représentée la bande continue d'obturation du passage des vapeurs diffusées par la plaquette qui est logée á l'intérieur de la boîte.

Dans la boîte support améliorée du type décrit et qui correspond au modèle d'utilité ES-U-289.919 du demandeur est supprimée la formation de créneaux et de ce fait, la boite comporte un corps á paroi latérale, annulaire 2 haute et continue qui détermine la hauteur á partir du fond 3 de la boîte jusqu'au bord de son embouchure d'entrée 4.

Sur la paroi verticale continue 2 de la boîte sont formées, en deux points opposés de cette paroi continue et qui sont situés sur les deux côtés latéraux opposés de la boîte, une multiplicité d'ouvertures rectangulaires 5, allongées, á petits côtés courbes, qui, á leurs extrémités, n'atteignent ni le fond 3 de la boîte ni le bord opposé libre de l'embouchure 4 permettant d'avoir accès á l'intérieur de la boîte.

Sur chaque côté de chacun des deux groupes, se faisant face, des ouvertures allongées précitées 5 se trouve une nervure 6 faisant saillie á partir de la surface interne de la paroi, chaque nervure 6 étant parallèle au bord de l'ouverture correspondante dont elle est voisine.

Une petite fenêtre 7 est percée á côté de chacune des nervures, en étant séparée de cette nervure.

Le couvercle 8 comporte un bord continu 9 s'étendant vers le bas, de même forme que l'embouchure de la boite. Ce bord a une extrémité inférieure en biseau 10. A partir du bord continu, qui forme une saillie interne 9 en forme de cadre, á partir de la surface interne du couvercle 8, sont formés des crochets juxtaposés et solidaires 11 permettant l'accrochage du couvercle dans les fenêtres correspondants 7 du corps de la boite.

La forme du cadre coïncide avec celle de l'embouchure 4 de l'ouverture de la boîte 1.

Les positions des extrémités libres souples des crochets 11 du couvercle correspondent, lorsque le couvercle est emboîté dans l'embouchure de la boîte, á celles des fenêtres 7 du corps de la boîte.

Les fenêtres 7 du corps de la boîte sont situées au voisinage du bord de son embouchure.

Lorsqu'on engage le couvercle 8 dans l'embouchure 4 d'entrée dans la boîte, et qu'on applique une pression d'introduction du bord périphérique 9 dans l'embouchure d'entrée dans la boîte, introduction qui est facilitée par l'extrémité en biseau 12, les extrémités libres souples des crochets cèdent vers l'arrière par suite de la poussée de la paroi interne du corps de la boîte qui, par suite de sa présence, les repousse sensiblement vers l'arrière, et en poursuivant le mouvement de descente du couvercle dans l'embouchure de la boîte, les crochets souples 11 atteignent les ouvertures des fenêtres correspondants 7 qui, par suite de l'absence de matière de la paroi á cet endroit, permettent aux crochets souples, qui étaient soumis á une compression, de reprendre leur position naturelle verticale, á l'état de repos. Le crochet pénétrant dans la fenêtre 7 accroche, par son côté supérieur de la fenêtre, en produisant ainsi de cette façon l'accrochage automatique de chaque crochet 11 dans sa fenêtre correspondante 7, en empêchant que le couvercle puisse se détacher par hasard du corps de la boîte.

Les fenêtres 7 sont situées au voisinage du bord de l'embouchure du corps de la boîte de telle façon que de ce fait, les jours qui peuvent exister entre le bord de chaque fenêtre 7 et le corps de chaque crochet engagé 11, peuvent être obturés par la même bande continue (non représentée) obturant les ouvertures des deux grands côtés latéraux de la paroi continue que forme le corps de la boîte.

Le bord de l'embouchure d'entrée de la boîte a en totalité une forme de biseau, sensiblement incliné de l'extérieur vers l'intérieur, afin de faciliter l'accouplement du bord périphérique 9 du couvercle 8 dans l'embouchure de la boîte.

Il doit être entendu que dans le cas présent, Qn peut envisager diverses variantes en ce qui concerne les détails de construction et de finition sans altérer ou modifier en quoi que ce soit l'essentiel de l'invention.

## Revendications

1. Boîte support améliorée pour des plaquettes diffusant des vapeurs et effluves odorants, désinfectants et insecticides, du type constitué de deux demi-boîtes oblongues (8,2), moulées en matière thermoplastique, à petits côtés courbes, avec des bandes s'étendant á partir de la base de chaque demi-boite, ces bandes se terminant en arc en plein-cintre convergent, les demis-boîtes se faisant face par leurs embouchures, l'extérieur des demi-boîtes étant pourvu, pour régler le passage des vapeurs suivant le désir de la personne utilisant de telles boîtes supports, d'une bande annulaire pourvue d'ouvertures dans sa surface adossée á la paroi latérale continue de la boîte allongée ainsi formée, paroi latérale continue dans des tronçons de laquelle sont formées des ouvertures (5) établissant une communication avec la partie interne de la boîte, le glissement latéral de la bande annulaire entraînant l'ouverture ou la fermeture du passage des ouvertures et de ce fait ouvrant ou fermant le passage pour les vapeurs de la plaquette émettrice de vapeur contenue á l'intérieur de la boîte, **caractérisée en ce que** le corps du support de ce type, fait de deux demi-boîtes, est formé d'une boîte et de son couvercle, **en ce que** les tronçons de la paroi continue correspondant aux grands côtés latéraux présentent une multiplicité d'ouvertures (5) rectangulaires, à petits côtés courbes, parallèles et séparées d'une manière appropriée de telle façon que leurs extrémités n'atteignent ni le bord du fond de la boîte ni le bord de l'embouchure de cette boîte, **en ce que** des deux côtés de chacun des deux groupes d'ouvertures transversales, disposées face á face, sont formées des fenêtres (7) voisines du bord continu en biseau (12) de l'embouchure du corps de la boîte, **en ce que** sur le couvercle de l'embouchure du corps de la boîte est formée une saillie latérale descendante de faible hauteur, á bord taillé en biseau (10), faisant saillie á partir du fond du couvercle et de forme identique á celle de l'embouchure de la boîte, **en ce que** sur la saillie descendante latérale continue sont répartis, en étant distribués régulièrement et unis d'une manière permanente, autant de crochets (11) qu'il y a de fenêtres (7), ces crochets assumant la fonction de détentes de telle façon que, lorsque l'on engage le couvercle dans l'embouchure du corps de la boîte et on exerce une pression vers le bas sur le couvercle, en direction du corps de la boîte, les extrémités libres souples des crochets soient repoussées par compression vers l'intérieur, par la propre matière de la paroi du corps de la boîte, jusqu'à ce que, en poursuivant le mouvement de descente, les crochets s'engagent dans les fenêtres correspondantes en y pénétrant et s'y accrochant sur les bords des fenêtres respectives lorsque les extrémités libres des crochets reprennent leur position naturelle á l'état de repos.

2. Boîte support améliorée pour des plaquettes diffusant des vapeurs et effluves odorants, désinfectants et insecticides suivant la revendication antérieure, **caractérisée en ce que** de chaque côté de l'un et l'autre groupe d'ouvertures transversales de la paroi continue qui forme les deux grands côtés latéraux du corps de la boîte, fait saillie, par rapport á la surface interne de la paroi, une nervure (6) parallèle aux ouvertures transversales et dont les extrémités rejoignent presque le bord de l'embouchure de la boîte et le fond de celle-ci.

3. Boîte support améliorée pour des plaquettes diffusant des vapeurs et effluves odorants, désinfectants et insecticides suivant les revendications antérieures, **caractérisée en ce qu'**elle comprend quatre fenêtres d'accrochage (7) et que pour obturer les jours qui subsistent entre les bords desdites quatre fenêtres d'accrochage (7) et les crochets (11) introduits dans ces fenêtres, ces fenêtres sont disposées au voisinage du bord de l'embouchure de la paroi continue du corps de la boîte de telle façon que la bande continue obturant les ouvertures transversales formées dans les deux côtés opposés de la paroi continue qui constitue le corps de la boîte, obture également ces jours laissés par l'introduction des crochets dans les fenêtres.

## Claims

1. Improved support casing for tablets diffusing odoriferous, disinfecting and insecticidal vapours and emissions, of the type constituted by two oblong semi-casings (8, 2), moulded from thermoplastic material and having curved short sides, with strips extending from the base of each semi-casing, those strips terminating in a converging full-centre arch, the semi-casings facing one another at their mouths, the outside of the semi-casings being provided, in order to adjust the passage of the vapours as desired by the person using such support casings, with an annular strip provided with openings in its surface resting against the continuous lateral wall of the elongate casing so formed, in portions of which continuous lateral wall are formed openings (5) forming a communication with the inner portion of the casing, the lateral sliding of the annular strip bringing about the opening or the closing of the passage of the openings and therefore opening or closing the passage for the vapours of the vapour-emitting tablet contained inside the casing, **characterised in that** the body of the support of this type, made of two semi-casings, is formed from a casing and its lid, **in that** the portions of the continuous wall corresponding to the long lateral sides have a multiplicity of rectangular openings (5) that have curved short sides and that are parallel and separated in an appropriate manner so that their ends reach neither the edge of the base of the casing nor the edge of the mouth of the casing, **in that** windows (7) adjacent to the bevelled continuous edge (12) of the casing body mouth are formed on the two sides of each of the two groups of transverse openings which face one another, **in that** there is formed on the lid of the casing body mouth a descending lateral projection, having a low height and a bevelled edge (10), which projects from the base of the lid and is in a form identical to that of the mouth of the casing, **in that** as many hooks (11) as there are windows (7) are dispersed over the continuous lateral descending projection, being regularly distributed and joined in a permanent manner, those hooks acting as detents in such a manner that, when the lid is engaged in the casing body mouth and pressure is exerted downwards on the lid, in the direction towards the casing body, the flexible free ends of the hooks are pushed back by compression towards the inside by the material belonging to the casing body wall until, by continuing the descending movement, the hooks engage in the corresponding windows by penetrating into them and hooking onto the edges of the respective windows when the free ends of the hooks re-assume their natural position in the resting state.

2. Improved support casing for tablets diffusing odoriferous, disinfectant and insecticidal vapours and emissions according to the preceding claim, **characterised in that**, from each side of the one and the other group of transverse openings of the continuous wall which forms the two long lateral sides of the body of the casing, there projects, relative to the inner surface of the wall, a rib (6) which is parallel with the transverse openings and the ends of which almost join the edge of the mouth of the casing and the base of the casing.

3. Improved support casing for tablets diffusing odoriferous, disinfectant and insecticidal vapours and emissions according to the preceding claims, **characterised in that** it comprises four hooking windows (7) and **in that**, in order to close off the gaps which remain between the edges of the said four hooking windows (7) and the hooks (11) introduced into those windows, the windows are disposed in the vicinity of the edge of the mouth of the continuous wall of the casing body in such a manner that the continuous strip closing off the transverse openings formed in the two opposite sides of the continuous wall which constitutes the body of the casing, also closes off those gaps left by the introduction of the hooks into the windows.

## Patentansprüche

1. Verbesserter Trägerbehälter für wohlriechende, desinfizierende und insektizide Dämpfe und Ausdünstungen verbreitende Plättchen, vom Typ, bestehend aus zwei länglichen Halbbehältern (8,2), die aus einem thermoplastischen Material gebildet und an den Schmalseiten gekrümmt sind, mit Streifen, die sich von der Basis jedes Halbbehälters erstrecken, wobei die Streifen bogenförmig in einem konvergierenden Rundbogen enden und die Halbbehälter mit ihren Mündungen einander gegenüberliegen, wobei die Außenseite der Halbbehälter zur Regelung des Durchgangs der Dämpfe gemäß dem Wunsch der solche Trägerbehälter benutzenden Person mit einem ringförmigen Streifen ausgestattet ist, der mit Öffnungen in seiner Oberfläche versehen ist, welche an der Seitenwand anliegt, die sich vom solcherart geformten, verlängerten Behälter fortsetzt, und in den Abschnitten der durchgehenden Seitenwand Öffnungen (5) gebildet sind, welche eine Verbindung mit dem Innenteil des Behälters herstellen, wobei die seitliche Verschiebung des ringförmigen Streifens das Öffnen oder Schließen des Durchgangs der Öffnungen zur Folge hat und dadurch den Durchgang für die Dämpfe des im Inneren des Behälters enthaltenen, dampfverströmenden Plättchens öffnet oder schließt, **dadurch gekennzeichnet, dass** der Trägerkörper jenes Typs, der aus zwei Halbbehältern besteht, aus einem Behälter und dessen Deckel gebildet ist, dass die großen Längsseiten entsprechenden Abschnitte der durchgehenden Wand eine Vielfalt von rechteckigen Öffnungen (5) aufweisen, die an den Schmalseiten gekrümmt, parallel und auf eine derart geeignete Weise getrennt sind, dass ihre äußersten Enden weder den Rand des Bodens des Behälters, noch den Rand der Mündung dieses Behälters erreichen, dass auf beiden Seiten jeder der beiden Gruppen von transversalen, einander gegenüberliegenden Öffnungen Fenster (7) gebildet sind, und zwar benachbart dem Rand, der sich mit abgeschrägter Kante (12) von der Mündung des Behälterkörpers fortsetzt, dass auf dem Deckel der Mündung des Behälterkörpers ein seitlicher Vorsprung gebildet ist, der mit geringer Höhe hinabführt, am Rand mit einer schrägen Kante (10) zugeschnitten ist, vom Boden des Deckels vorragt und eine Form aufweist, die mit jener der Mündung des Behälters identisch ist, dass auf dem nach unten gerichteten, durchgehenden, seitlichen Vorsprung ebenso viele Haken (11), wie es Fenster (7) gibt, verteilt sind, wobei sie regelmäßig verteilt und permanent verbunden sind und die Haken die Funktion von Abzugshebeln annehmen, und zwar in einer solchen Weise, dass, wenn der Deckel in die Mündung des Behälterkörpers eingesetzt und nach unten, in Richtung des Behälterkörpers, auf den Deckel Druck ausgeübt wird, die freien, biegsamen Enden der Haken mittels Druck nach innen, durch das besondere Material der Wand des Behälterkörpers, zurückgedrückt werden, bis die Haken bei Beibehaltung der Abwärtsbewegung mit den entsprechenden Fenstern in Eingriff kommen, indem sie dort eindringen und an den Rändern der jeweiligen Fenster hängen, wenn die freien Enden der Haken im Ruhezustand ihre natürliche Position wieder einnehmen.

2. Verbesserter Trägerbehälter für wohlriechende, desinfizierende und insektizide Dämpfe und Ausdünstungen verbreitende Plättchen gemäß dem vorhergehenden Anspruch, **dadurch gekennzeichnet, dass** von jeder Seite der einen und der anderen Gruppe von transversalen Öffnungen der durchgehenden, die beiden großen Längsseiten des Behälterkörpers bildenden Wand im Verhältnis zur Innenfläche der Wand eine zu den transversalen Öffnungen parallele Rippe (6) vorspringt, deren Enden beinahe den Rand der Mündung des Behälters und dessen Boden erreichen.

3. Verbesserter Trägerbehälter für wohlriechende, desinfizierende und insektizide Dämpfe und Ausdünstungen verbreitende Plättchen gemäß den vorhergehenden Ansprüchen, **dadurch gekennzeichnet, dass** er vier Einhängefenster (7) umfasst und dass zwecks Verschließens der Öffnungen, welche zwischen den Rändern der besagten vier Einhängefenster (7) und den in diese Fenster eingeführten Haken (11) existieren, die Fenster in der Nähe des Rands der Mündung der durchgehenden Wand des Behälterkörpers solcherart angeordnet sind, dass der durchgehende Streifen, der die transversalen Öffnungen verschließt, welche in den beiden gegenüberliegenden Seiten der den Behälterkörper bildenden, durchgehenden Wand gebildet sind, gleichermaßen die für die Einführung der Haken in die Fenster belassenen Öffnungen verschließt.
